# EUROPEAN PATENT SPECIFICATION

(11) **EP 1 377 182 B1**
(45) Date of publication and mention of the grant of the patent: **16.08.2006**
(21) Application number: 02735202.0
(22) Date of filing: 30.03.2002
(51) Int. Cl.: A23L 1/30, A61K 8/97

(54) **USE OF LIGNANS IN FOODS**
VERWENDUNG VON LIGNAN IN NAHRUNGSMITTELPRODUKTEN
UTILISATION DE LIGNANS DANS DES ALIMENTS

(30) Priority: 04.04.2001 EP 01303208
(43) Date of publication of application: 07.01.2004
(73) Proprietor: UNILEVER N.V., 3013 AL Rotterdam (NL); UNILEVER PLC, London, Greater London EC4P 4BQ (GB)
(72) Inventor: CASSIDY, Aedin, Bedford,MK44 3JJ (GB); GREEN, Martin Richard, Bedford, Bedfordshire MK44 1LQ (GB); RICHARDS, Mark, Stanmore, Middlesex HA7 3SA (GB); TASKER, Maria Catherine, Bedford, Bedfordshire MK44 1LQ (GB)
(74) Representative: Hugot, Alain
(86) International application number: PCT/EP2002/003585
(87) International publication number: WO 2002/080702

(56) References cited:
- WO-A-99/07239
- US-A- 5 762 935
- US-A- 5 780 060
- US-A- 5 837 256
- US-A- 6 039 955
- DATABASE MEDLINE [Online] October 2000 (2000-10) OWEN R W ET AL: "Olive-oil consumption and health: the possible role of antioxidants." Database accession no. NLM11905662 XP002211169 & THE LANCET ONCOLOGY. ENGLAND OCT 2000, vol. 1, October 2000 (2000-10), pages 107-112, ISSN: 1470-2045
- DATABASE WPI Section Ch, Week 199901 Derwent Publications Ltd., London, GB; Class B02, AN 1999-005147 XP002211170 & JP 10 279461 A (NISSHIN OIL MILLS LTD), 20 October 1998 (1998-10-20)

## Description

### FIELD OF THE INVENTION

The present invention relates to foods with anti-inflammatory and/or anti-ageing properties.

### BACKGROUND OF THE INVENTION

Lignans are well known compounds which are present in a number of natural products such as flaxseeds, tea and coffee (see for example EP-A-906 761 and British Journal of Nutrition, volume 79 (1998) pages 37-45 "Lignan and isoflavonoid concentrations in tea and coffee").

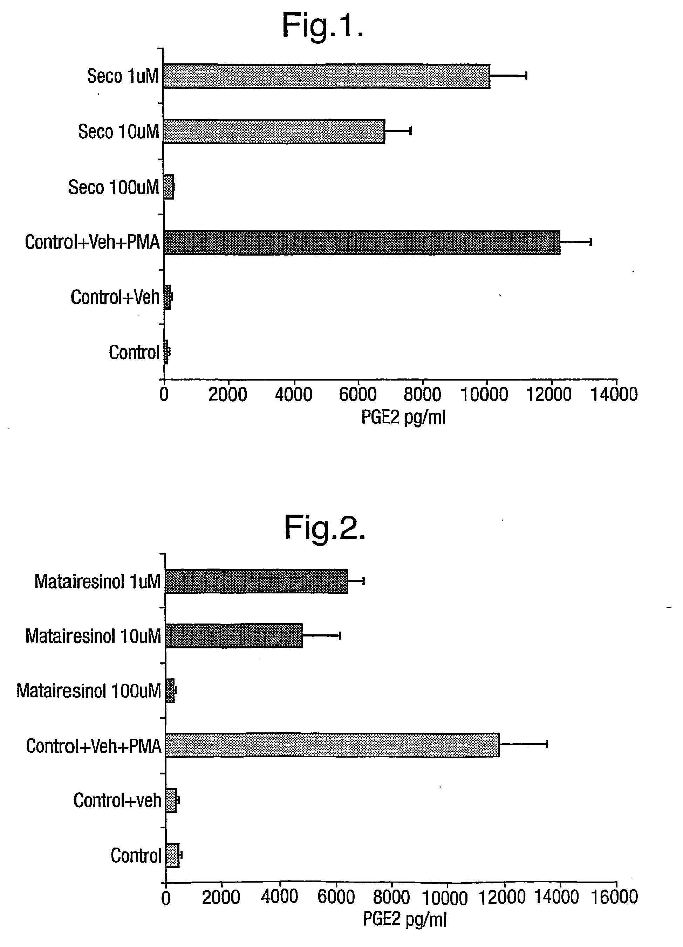

Examples of lignans are secoisolariciresinol (SECO) and matairesinol (MAT). Lignans are 2,3 dibenzylbutane derivatives and the chemical structure for SECO and MAT is represented in Fig 1 of the above British Journal of Nutrition. It is further known that SECO and MAT can be the precursors of enterolactone and enterodiol and modifications thereof including diglucosides (see EP-A-906 761, page 2 lines 53-56).

A number of health effects are known for lignans. EP-A-906 761 discloses the use of lignans for the prevention of a number of cancer diseases, reducing hot flashes, preventing osteoporosis, anti viral activities, antimitotic activity and fungicidal activity. WO 00/13661 discloses the use of lignans in topical compositions for guarding against bruising of the skin. WO 99/07239 proposes to use lignans derived from flaxseed in a functional food preparation intended to alleviate problems related with menopausal women. WO 00/19842 discloses that an abrased fraction of bleached flaxseed (rich in lignans) can be used for achieving taste or structural or colour effects in food products(see page 6, lines 29-31) and/or to increase the fibre intake of the person typically consuming such foods. No disclosure is given of any health benefits.

US 5,762,935 discloses the use of lignans of the sesamin family to treat infection and inflammation by delivering these lignans either enterally or parenterally especially as a total parenteral nutrition solution or as a dietary supplement.

US 5 837 256 discloses the use of secoisolariciresinol and secoisolariciresinol diglucoside in a substantially pure form for the treatment of Lupus nephritis, an auto-immune disease in which the patients immune system attacks his/her own organs. No disclosure is given that these lignans are effective when used in anything other than a substantially pure form.

US 6,039,955 discloses the lignan nordihydroguaiaretic acid (NDGA) in an agent for the treatment of inflammation. The agent may be a pill, capsule or a powder.

In EP-A-038 600 and EP-A-043 150 synthetic compounds are disclosed that have a chemical structure similar to that of SECO and MAT (but having only one substituant in the phenyl groups instead of two as is the case with SECO and MAT). The compounds disclosed in EP-A-038 600 and EP-A-043 150 are said to possess anti-inflammatory properties but are used in pharmaceutical compositions only and not in foods (probably due to the fact that these compounds are not natural compounds).

Biological effects including an increase in the excretion of the metabolites enterodiol and enterolactone have been observed when healthy postmenopausal women, ages 52-82 years, consumed their habitual diets plus 0, 5, or 10 grams of ground flaxseed per day (Cancer Epidemiol Biomarkers Prev 2000 Oct;9(10):1113-8).

However the prior art does not disclose other beneficial health effects such as anti-ageing effects, in particular anti-ageing effects of the skin, for lignans.

Also not disclosed in the prior art are food products with anti-inflammatory benefits and comprising lignans derived from flaxseed, enterolactone, enterodiol and precursors thereof, and especially secoisolariciresinol (SECO) and matairesinol (MAT).

There is now emerging an increasing desire for food products to provide more than simply a pleasing taste, or, basic nutritional value to the consumer of the product. Both consumers and the food industry are starting to seek additional benefits from their food products in order to improve, or maintain, the general health and well-being of people consuming such food products.

By "nutritional value" is meant the vitamin and mineral content of the food product. This is sometimes referred to in the art as the micro-nutrient value of the food product. Additional benefits which are now being sought from food products include amongst others, anti-inflammatory benefits and anti-ageing benefits. Such foods are sometimes termed "functional foods".

A further problem is that such food products do not always have acceptable physical parameters or characteristics such as stability (antioxidant properties), improved taste (intrinsic flavour) and improved physical structure (cross-linking). This can be a problem especially for food products which comprise an appreciable amount of fat.

Accordingly, there is a need in the art to provide food products which provide benefits to improve, or maintain, the general health and well-being of a person regularly consuming such a food product. Such food products desirably also have a pleasing taste and/or are nutritious.

Furthermore, there is also a need to provide food products which have good physical parameters or characteristics such as those mentioned hereinabove.

The present invention seeks to address one or more of the above problems.

The terms SECO and MAT as used hereinafter refer respectively to secoisolariciresinol and matairesinol.

### SUMMARY OF THE INVENTION

We have surprisingly found that lignans have anti-ageing effects upon the human body, in particular upon the skin which can result in improved appearance thereof, and can be included in food products to provide these benefits to the consumer thereof.

We have also surprisingly found that anti-inflammatory benefits can be obtained from food products comprising lignans derived from flaxseed, enterolactone, enterodiol and precursors thereof, and especially secoisolariciresinol (SECO) and matairesinol (MAT) and that there is no need to use these compounds in a substantially pure form.

Without wishing to be bound by theory, it is believed that these effects occur due to the lignans promoting procollagen-1 production and reducing PGE2 production in the body.

Furthermore we have found that lignans can also be used to achieve beneficial effects upon a number of physical parameters or characteristics of food products, in particular, those which contain an appreciable amount of fat. These beneficial effects concern, in particular, improved stability (antioxidant properties). Other benefits include improved taste (intrinsic flavour) and improved physical structure (cross-linking).

Therefore our invention provides according to a first embodiment the use of one or more health components selected from the group consisting of lignans in the production of a food product with anti-ageing properties.

For the first aspect it is preferred that the health components are selected from lignans derived from flaxseed, enterolactone, enterodiol and precursors thereof.

According to the second aspect the invention provides the use of one or more health components selected from the group of consisting of lignans derived from flaxseed, enterolactone, enterodiol and precursors thereof in the production of a food product with anti-inflammatory properties.

It is especially preferred according to both the first and second aspects that the lignans are selected from secoisolariciresinol (SECO) and matairesinol (MAT).

The food product may have both anti-inflammatory properties and anti-ageing properties.

According to a third aspect, the present invention provides a method for administering a health component selected from the group consisting of lignans derived from flaxseed, enterolactone, enterodiol and precursors thereof, to humans in need of the intake of an anti-inflammatory component or of an anti-ageing component, wherein the human is administered an effective daily amount of the health component by feeding this human with a food product comprising of from 10 to 100 or 200 wt% of the recommended daily amount of the health component.

Typical levels of the active (health) component consumed (administered) would be in the range of from 45-100mg/day and for the purpose of this invention is considered as the recommended daily amount.

In particular secoisolariciresinol and matairesinol are administered according to the third aspect of the invention.

The lignans are preferably administered to a human in need of an anti-ageing component with an activity on ageing of the skin.

The "effective daily amount" administered to the human in need of the intake of an anti-inflammatory or an anti-ageing component is that amount which results in a noticeable improvement of the defect in question.

Where a mixture of lignans is used, the amounts referred to herein refer to the amount of that mixture.

A daily portion of the food product is that amount which is typically consumed in a day for the type of food product in question. It may be consumed in more than one sitting but is ideally consumed in one (a single daily portion) or two sittings.

Except in the operating and comparative examples, or where otherwise explicitly indicated, all numbers in this description indicating amounts of material or conditions of reaction, physical properties of materials and/or use are to be understood as modified by the word "about." All amounts are by weight, unless otherwise specified.

### DETAILED DESCRIPTION OF THE INVENTION

The invention will now be discussed in greater detail.

Lignans other than secoisolariciresinol and matairesinol which can be used according to the present invention are selected from the group consisting of plicatic acid; pinoresinol; lariciresinol; podophyllotoxin; trachelogenin; shonanin and enterofuran (see Xia ZQ c.s in J Biol Chem 2001,Jan 18,PMID 11278426 and Liggins c.s in Analytical Biochem,287,2000,p.102-109).

The levels of the health component (lignans) used in the food product can vary considerably but it is demonstrated that levels sufficient to provide of from 10 to 100 or 200 wt%, for example 20 to 95wt%, of the recommended daily amount of the health component upon consumption of a daily portion of the food product can easily be used according to the invention.

Typically the lignans are used at a level sufficient to provide of from 0.01 to 95 wt% of the lignans in the food product (by weight of the food product), preferably of from 0.5 to 35 wt%, more preferably of from 0.8 to 10 wt%. For some food products lower levels within these ranges may be preferred, such as 0.02 to 5 wt%, preferably 0.05 to 2.5 wt%.

The lignans can be added to, and incorporated in, the food product in any suitable delivery form including; as a free compound, as a concentrate or an extract of a natural product, in particular as a concentrate of flaxseed, in encapsulated form (in particular encapsulated by a sugar, a starch or gelatin), or, as a powder or crystals. If a powder or crystals are used it is preferably used on a carrier.

The lignans may be added to the food product (or components thereof) by any suitable method known in the art, for example by mixing with a blade mixer or other known food mixers.

The lignans may be incorporated into many types of food products for all aspects of the invention. Typical examples include spreads, dressings, mayonnaises, ice creams, cream alternatives, health bars, health drinks, sports drinks, chocolates, confectionery, bakery products, soups, cereals, sauces, fillings and coatings.

According to the third aspect of the invention, the actual amount of lignans administered will depend upon the type of food product, the delivery form used and the type of health deficiency. The lignans may be administered by eating one or more food products or portions thereof.

Moreover it is believed that lignans can also be used to synergistically enhance health effects of other natural health components, especially the anti-inflammatory or anti-ageing effects of other natural anti-inflammatory or anti-ageing components in food products. For example combinations of our lignans with isoflavones or flavones may demonstrate such a synergy in the health effects known for these isoflavones and flavones only. Such other isoflavones or other flavones may be used in the food product in any suitable amount.

Our lignans may also be used in combination with other micronutrients, for example vitamins such as vitamin C and vitamin E.

As indicated above the lignans may also have a beneficial effect on the physical performance/ characteristics of food products. In particular the use of the lignans in food products, especially in the amounts mentioned above, can result in improvements in product quality especially in stability (antioxidant properties). Other benefits include taste (intrinsic flavour) and physical structure (cross-linking) of the food product.

The present invention will be further explained with reference to the following non-limiting examples.

### EXAMPLES

### Example 1 - Anti-inflammatory effects; Procedure for measuring PGE2 levels in human dermal fibroblasts.

### Anti-inflammatory cell assays.

It is emphasized that the anti-inflammatory effects were determined by in vitro tests wherein the Prostaglandin E2 (=PGE2) production by the human skin fibroblasts is measured after being induced by the inflammatory modulus phorbyl myristyl acetate (PMA). A reduction of the levels of PGE2 is indicative of the anti-inflammatory effect.

Fibroblast cell assay: Primary human foreskin fibroblasts at passage 2 (P2) were seeded into 96-well plates at 35000 cells/well and maintained for 24 hours in an atmosphere of 5% carbon dioxide in Dulbeccos Modified Eagles Medium (DMEM) supplemented with 10% foetal calf serum.

The lignan-compound (SECO or MAT) was added to fresh cell media (DMEM, supplemented with 10% foetal calf serum) in 100% ethanol (final concentration 1%) in triplicate and incubated for a further 24 hours. Phorbal myristate acetate (PMA) in ethanol/cell media (10nm) was added to the cells treated with the lignan compound (SECO) and the cells incubated for a further 24 hours. PMA represents an external stressor, which induces oxidative stress and inflammatory responses in cells. The fibroblasts/ media were then analysed as described below immediately, or, snap frozen in liquid nitrogen and stored at - 70°C for future analysis. The cells were then counted to ensure no effect on cell number.

Different concentrations of SECO or MAT were used in the above method to study the effect of concentration. A series of control experiments were also carried out according to the above method; Control + veh + PMA (positive control), Control + veh (negative control) and control (standard control).

Prostaglandin E2 (PGE2) assay volumes of 50 µl culture medium were taken for PGE2 assay from the above samples after gently shaking the culture plate. PGE2 levels in the medium were determined with a Biotrak PGE2 immunoassay kit (Amersham, UK). The assay is based on the competition between unlabelled PGE2 in the sample and a fixed quantity of horseradish peroxidase labelled PGE2 for a limited amount of fixed PGE2 specific antibody. Concentrations of unlabelled sample PGE2 are determined according to a standard curve, which was obtained at the same time.

The results of the administering of different amounts of SECO or MAT are illustrated in respectively figures 1 and 2. The results show that SECO and MAT are effective in producing an anti-inflammatory effect in human cells as can be seen by comparing the result for the control + veh + PMA with the results obtained for the three different levels of Isoxanthohumol. Increasing concentrations of SECO and MAT produced increasing anti-inflammatory effects as measured by a reduction in PGE2.

### Example 2 - Anti-ageing effects; Procedure For Measuring Procollagen-I and Decorin Synthesis In Hucnan Dermal Fibroblasts.

### Anti-ageing cell assays; preparation of dermal fibroblast conditioned medium.

It is emphasized that the anti-ageing effects were determined by in vitro tests wherein the Procollagen-I and Decorin production by the human dermal fibroblasts is measured after being induced by the anti-ageing stimulus. An increase in the levels of Procollagen-I is indicative of the anti-ageing effect.

Preparation of Dermal Fibroblast Conditioned Medium: Primary human foreskin fibroblasts at passage 2 (P2) were seeded into 12-well plates at 40000 cells/well and maintained for 24 hours in an atmosphere of 5% carbon dioxide and 4% oxygen in Dulbeccos Modified Eagles Medium (DMEM) supplemented with 10% foetal calf serum. After this time the cells were washed with serum free DMEM and then incubated in fresh serum free DMEM for a further 60 hours. The fibroblast monolayers were then washed again with serum free DMEM.

The test reagent (MAT and SECO at different concentration levels) and vehicle control (1% ethanol final concentration) were added to the cells in triplicate in a final volume of 0.4 ml/well fresh serum free DMEM and incubated for a further 24 hours. This fibroblast conditioned medium was either analysed immediately, or, snap frozen in liquid nitrogen and stored at - 70°C for future analysis. The cells were then counted and data from the dot-blot analysis subsequently standardised to cell number.

Dot Blot Assay for Decorin Protein in Dermal Fibroblast Conditioned Medium: Samples of conditioned medium from dermal fibroblasts treated with vehicle (as a control) or test reagent were supplemented with 20mM dithiothreitol (1:10 dilution of 200mM stock solution) and 0.1% sodium dodecylsulphate (1:100 dilution of 10% stock solution), mixed well and then incubated at 75°C for 2 minutes.

A standard for the assay was generated by serial dilution of neat fibroblast conditioned medium from fibroblasts seeded at 10000 cells/cm² in a 175cm² flask and maintained in serum free DMEM as described above.

Assay samples were subsequently applied in triplicate to a pre-wetted sheet of Immobilon-P transfer membrane using the 96-well Bio-Dot Apparatus from Bio-Rad as described in the manufacturer's guidelines. Approximately 200µl of medium was applied per well. The medium was allowed to filter through the membrane under gravity (30 minutes) after which the membrane was washed twice with PBS (200µl). These PBS washes were allowed to filter through the membrane under gravity (2x15 minutes). The Bio-Dot apparatus was then attached to a vacuum manifold and a third and final PBS wash carried out under suction. The apparatus was disassembled, the membrane removed and quickly cut as required before being placed in blocking buffer overnight at 4°C.

Membranes prepared for decorin analysis were blocked with 3% (w/v) bovine serum albumin (BSA)/ 0.1% (v/v) Tween 20 in phosphate buffered saline (PBS), whilst those for procollagen-I analysis were blocked with 5% (w/v) non fat dried milk powder/0.05% Tween 20 in PBS. The following day, the membranes were probed with 1:10000 dilution of primary antibodies to human decorin (rabbit polyclonal; Biogenesis) for 2 hours at room temperature. The membranes were subsequently washed with TBS/0.05% Tween 20 (3 x 15 minutes) and then incubated with 1:1000 dilution of ¹²⁵I-conjugated anti-rat or anti-rabbit F(ab')2 fragments (Amersham) as required for 1 hour at room temperature. Following this the Immobilon strips were again washed with TBS/Tween 20 (3 x 15 minutes) before being allowed to dry in air at room temperature. The dried membranes were wrapped in cellophane and exposed to a Molecular Dynamics storage phosphor screen for 16-18 hours. At the end of this time the exposed screen was scanned by a phosphorimager (Molecular Dynamics Phosphorimager SF) using ImageQuant^{™} software. Dot intensity was assessed by computer-assisted image analysis using the quantification tools in ImageQuant^{™}, standardised to cell number and the effects of various test reagents on decorin and procollagen-I synthesis were determined relative to a vehicle treated control value of 100 arbitrary units.

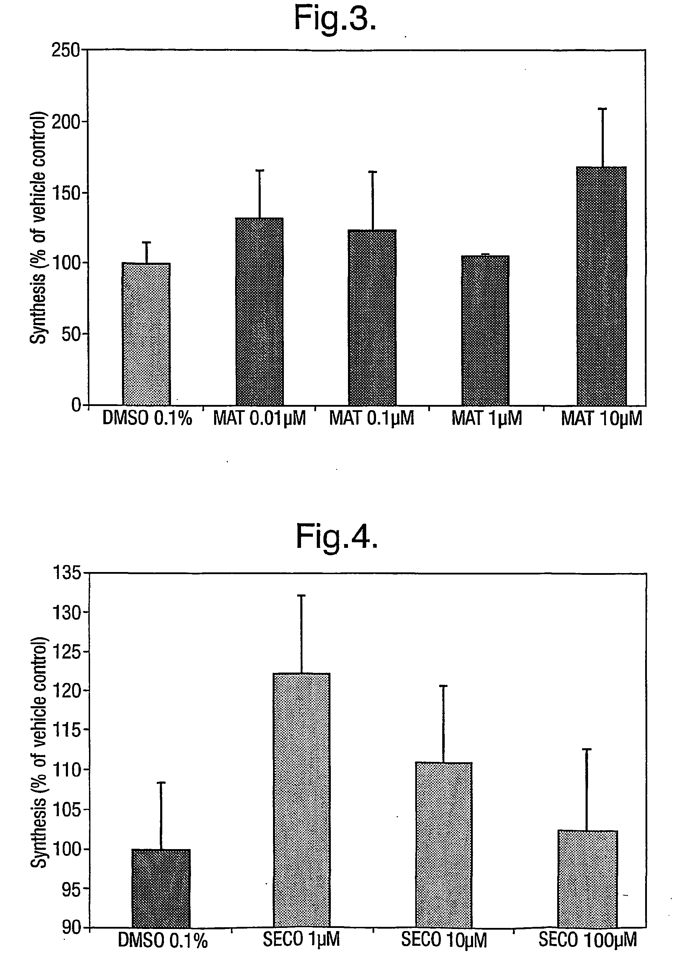

The results obtained for MAT and SECO are represented in figures 3 and 4 respectively. These results show that the MAT and SECO samples showed increased anti-ageing effects in human cells compared to a control sample.

### Example 3

An ice cream can be prepared according to the following recipe;

| | Wt% |
|---|---|
| SECO | 0.02 |
| Fat blend | 10.0 |
| Skimmed milk powder | 10.0 |
| Icing sugar | 12.0 |
| Corn syrup solids | 4.0 |
| Dextrose monohydrate | 2.0 |
| Sherex IC 93300 | 0.6 |
| Water to balance of | 100.0 |

Sherex IC 93300 is a product comprising mono- and diglycerides mixed with different stabilizers from Quest International.

Mix the ingredients except the water and the fat blend. Add the water (cold) to this mixture and then heat in a water bath to a temperature of 70°C. Add the fat blend (fully liquid palm oil) whilst the mixture is 'stirred' in a high speed mixer e.g. an Ultra-turax mixer to form an emulsion. Cool the mixture in a water bath to 20°C and then stir again e.g. in the ultra-turrax.

Place the emulsion in a batch ice cream making machine (held for 24 hours at -28°C prior to use) and stir for 15 minutes.

An ice cream of good quality is obtained. The manufacture of the above ice cream can be repeated using MAT instead of SECO.

### Example 4

A spread containing SECO may be prepared from the following ingredients using the process below:

| | wt% |
|---|---|
| SECO | 5x10⁻³ |
| Oil blend | 49.5 |
| Lecithin | 0.205 |
| distilled monoglyceride | 0.3 |
| Flavour | 0.01 |
| Colour | 0.0066 |
| Whey | 0.25 |
| EDTA | 0.007 |
| Citric Acid | 0.03 |
| K Sorbate | 0. 1 |
| Salt | 1.6 |
| Water to balance of | 100.0% |

Mix the fat and aqueous phases together at approximately 55°C in a heated tank in a ratio of approximately 40 parts fat phase to 60 parts aqueous phase to produce a fat continuous emulsion. The aqueous phase is added to the fat phase to aid in obtaining a fat continuous emulsion.

Pass the emulsion through a cooled, scraped-surface heat exchanger (A-unit) where the emulsion is cooled to a temperature at which the fat will begin to crystallize (about 8-20°C.) and the aqueous phase will begin to increase in viscosity. Pass the cooled emulsion through a C-unit, crystallizer; the shaft speed may vary and depends upon it's dimensions and the residence time required to crystallize the fat. Typical speeds are in the range of from 100-900 RPM. Pass the fat continuous emulsion into an additional cooling unit to reduce the temperature of the emulsion as there is a temperature rise due to heat of crystallization in the crystallizer. For a tub packaged product, the cooled emulsion is passed through the crystallizer (C-Unit) to provide additional residence time and adjust the consistency for packaging in tub. For a stick or bar product, pass the cooled emulsion through a B-Unit for additional residence time and to increase the packing hardness for the product to be packed in the stick or bar form.

A spread of good quality is obtained. The manufacture of the spread of this example can be repeated using MAT instead of SECO.

## Claims

1. The use of one or more health components selected from the group of consisting of lignans in the production of a food product with anti-ageing properties.

2. The use according to claim 1 wherein the one or more health components are selected from lignans derived from flaxseed, enterolactone, enterodiol and precursors thereof.

3. The use of one or more health components selected from the group of consisting of lignans derived from flaxseed, enterolactone, enterodiol and precursors thereof in the production of a food product with anti-inflammatory properties.

4. The use according to either claim 2 or claim 3 wherein the one or more health components are selected from secoisolariciresinol and matairesinol.

5. The use according to any one of claims 1 to 4 wherein the one or more health components are used at a level sufficient to provide of from 10 to 200 % of the recommended daily amount of the health component upon consumption of a daily portion of the food product.

6. The use according to claim 5 wherein the one or more health components are used at a level sufficient to provide of from 10 to 100 wt% of the recommended daily amount.

7. The use according to any one of claims 1 to 6 wherein the one or more health components are used in the production of a food product at a level sufficient to provide of from 0.01 to 95 wt% of the one or more health components in the food product by weight thereof.

8. The use according to claim 7 wherein the one or more health components are used at a level sufficient to provide of from 0.02 to 5 wt% thereof in the food product.

9. The use according to any one of claims 1 to 8 wherein the food product is selected from the group consisting of spreads, dressings, mayonaisses, ice creams, cream alternatives, health bars, health drinks, sports drinks, chocolates, confectionery, bakery products, soups, cereals, sauces, fillings and coatings.

10. The use according to any one of claims 1 to 9 wherein the one or more health components are used to achieve stabilisation of the food product.

11. The use according to any one of claims 1 to 10 wherein the one or more health components are incorporated as a free compound, or as a concentrate or an extract of a natural product, or in encapsulated form, or as a powder or crystals.

12. The use according to claim 11 wherein the one or more health components are incorporated as a concentrate of flaxseed.

13. The use according to claim 11 wherein the one or more health components in encapsulated form are encapsulated in a sugar, a starch, or gelatin.

## Patentansprüche

1. Verwendung einer oder mehrerer Gesundheitskomponente(n), die aus der aus Lignanen bestehenden Gruppe ausgewählt ist (sind), in der Herstellung eines Nahrungsmittelproduktes mit "anti-ageing"-Eigenschaften.

2. Verwendung nach Anspruch 1, wobei die eine oder mehreren Gesundheitskomponente(n) aus Lignanen ausgewählt ist (sind), die von Leinsamen, Enterodiol und Vorstufen davon abgeleitet sind.

3. Verwendung einer oder mehrerer Gesundheitskomponente(n), die aus der Gruppe ausgewählt ist (sind), die aus Lignanen besteht, welche von Leinsamen, Enterodiol und Vorstufen davon abgeleitet sind, in der Herstellung eines Nahrungsmittelproduktes mit anti-inflammatorischen Eigenschaften.

4. Verwendung nach Anspruch 2 oder Anspruch 3, wobei die eine oder mehreren Gesundheitskomponente(n) aus Secoisolariciresinol und Matairesinol ausgewählt ist (sind).

5. Verwendung nach einem der Ansprüche 1 bis 4, wobei die eine oder mehreren Gesundheitskomponente(n) in einer Konzentration verwendet wird (werden), die ausreichend ist, um 10 bis 200 % der empfohlenen Tagesmenge der Gesundheitskomponente bei Verzehr einer Tagesportion des Nahrungsmittelproduktes bereitzustellen.

6. Verwendung nach Anspruch 5, wobei die eine oder mehreren Gesundheitskomponenten in einer Konzentration verwendet werden, die ausreicht, um 10 bis 100 Gew.-% der empfohlenen Tagesmenge bereitzustellen.

7. Verwendung nach einem der Ansprüche 1 bis 6, wobei die eine oder mehreren Gesundheitskomponente(n) in der Herstellung eines Nahrungsmittelproduktes in einer Konzentration, die ausreicht, um 0,01 bis 95 Gew.-% einer oder mehrerer Gesundheitskomponente(n) im Nahrungsmittelprodukt, bezogen auf das Gewicht, bereitzustellen.

8. Verwendung nach Anspruch 7, wobei die eine oder mehreren Gesundheitskomponente(n) in einer Konzentration verwendet wird (werden), die ausreicht, um 0,02 bis 5 Gew.-% davon im Nahrungsmittelprodukt bereitzustellen.

9. Verwendung nach einem der Ansprüche 1 bis 8, wobei das Nahrungsmittelprodukt aus der Gruppe, bestehend aus Aufstrichen, Dressings, Mayonnaisen, Speiseeis, alternativer Sahne, Süßwaren (Konfekt), Backwaren, Suppen, Cerealien, Soßen, Füllungen und Überzügen, ausgewählt wird.

10. Verwendung nach einem der Ansprüche 1 bis 9, wobei die eine oder mehreren Gesundheitskomponente(n) verwendet werden, um eine Stabilisierung des Nahrungsmittelproduktes zu erreichen.

11. Verwendung nach einem der Ansprüche 1 bis 10, wobei die eine oder mehreren Gesundheitskomponente(n) als freie Verbindung oder als Konzentrat oder Extrakt eines natürlichen Produktes oder in eingekapselter Form oder als Pulver oder Kristalle eingearbeitet ist (sind).

12. Verwendung nach Anspruch 11, wobei die eine oder mehreren Gesundheitskomponente(n) als ein Flachsamenkonzentrat eingearbeitet ist (sind).

13. Verwendung nach Anspruch 11, wobei die eine oder mehreren Gesundheitskomponente(n) in eingekapselter Form in einem Zucker, einer Stärke oder Gelatine eingekapselt ist (sind).

## Revendications

1. Utilisation d'un ou plusieurs composés de santé choisis dans le groupe constitué des lignanes dans la production d'un produit alimentaire avec des propriétés anti-vieillissement.

2. Utilisation selon la revendication 1 dans laquelle un ou plusieurs composés de santé sont choisis parmi les lignanes dérivées de la graine de lin, de l'entérolactone, de l'entérodiol et des précurseurs de ceux-ci.

3. Utilisation d'un ou plusieurs composés de santé choisis dans le groupe constitué des lignanes dérivées de la graine de lin, de l'entérolactone, de l'entérodiol et des précurseurs de ceux-ci dans la production d'un produit alimentaire avec des propriétés anti-inflammatoires.

4. Utilisation selon la revendication 2 ou la revendication 3 dans laquelle un ou plusieurs composés de santé sont choisis parmi le sécoisolaricirésinol et le matairésinol.

5. Utilisation selon l'une quelconque des revendications 1 à 4 dans laquelle un ou plusieurs composés de santé sont utilisés à une teneur suffisante pour fournir 10 à 200 % de la quantité quotidienne recommandée du composant diététique par la consommation d'une portion quotidienne du produit alimentaire.

6. Utilisation selon la revendication 5 dans laquelle un ou plusieurs composés de santé sont utilisés à une teneur suffisante pour fournir 10 à 100 % en poids de la quantité quotidienne recommandée.

7. Utilisation selon l'une quelconque des revendications 1 à 6 dans laquelle un ou plusieurs composés de santé sont utilisés dans la production d'un produit alimentaire à une teneur suffisante pour fournir 0,01 à 95 % en poids d'un ou de plusieurs composés de santé dans le produit alimentaire en poids de celui-ci.

8. Utilisation selon la revendication 7 dans laquelle un ou plusieurs composés de santé sont utilisés à une teneur suffisante pour fournir 0,02 à 5 % en poids de ceux-ci dans le produit alimentaire.

9. Utilisation selon l'une quelconque des revendications 1 à 8 dans laquelle le produit alimentaire est choisi dans le groupe constitué des produits à tartiner, des vinaigrettes, des mayonnaises, des crèmes glacées, des succédanés de crèmes, des barres pour la santé, des boissons pour la santé, des boissons sportives, des chocolats, des confiseries, des produits de boulangerie, des soupes, des céréales, des sauces, des garnissages et des enrobages.

10. Utilisation selon l'une quelconque des revendications 1 à 9 dans laquelle un ou plusieurs composés de santé sont utilisés pour réaliser la stabilisation du produit alimentaire.

11. Utilisation selon l'une quelconque des revendications 1 à 10 dans laquelle un ou plusieurs composés de santé sont incorporés sous la forme d'un composé libre, ou d'un concentré ou d'un extrait d'un produit naturel, ou sous forme encapsulée, ou sous forme d'une poudre ou de cristaux.

12. Utilisation selon la revendication 11 dans laquelle un ou plusieurs composés de santé sont incorporés sous la forme d'un concentré de graine de lin.

13. Utilisation selon la revendication 11 dans laquelle un ou plusieurs composés de santé sous la forme encapsulée sont encapsulés dans un sucre, un amidon, ou une gélatine.
